(19) Europäisches Patentamt
European Patent Office
Office européen des brevets

(11) Veröffentlichungsnummer: **0 272 667**
**A2**

(12) **EUROPÄISCHE PATENTANMELDUNG**

(21) Anmeldenummer: **87118928.8**

(51) Int. Cl.[4]: **C07H 9/02**, C07H 13/04

(22) Anmeldetag: **21.12.87**

(30) Priorität: **24.12.86 DE 3644375**
**08.01.87 DE 3700331**

(43) Veröffentlichungstag der Anmeldung:
**29.06.88 Patentblatt 88/26**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim (Taunus)(DE)**
Erfinder: **Grabley, Susanne, Dr.**
**Hölderlinstrasse 7**
**D-6240 Königstein/Taunus(DE)**
Erfinder: **Raether, Wolfgang, Dr.**
**Falkensteinstrasse 6**
**D-6072 Dreieich(DE)**
Erfinder: **Clommer, Bernd, Dr.**
**Berliner Ring 5**
**D-6233 Kelkheim (Taunus)(DE)**
Erfinder: **Kluge, Heinz, Dr.**
**Am Alten Birnbaum 10a**
**D-6238 Hofheim am Taunus(DE)**
Erfinder: **Sachse, Burkhard, Dr.**
**An der Ziegelei 30**
**D-6233 Kelkheim (Taunus)(DE)**

(54) **Verfahren zur Herstellung der Desmalonylverbindung von Makrolid-Lactonen.**

(57) Die Herstellung der Desmalonylverbindungen von Makrolid-Lactonen ist durch Hydrolyse in Gegenwart einer Base möglich, ohne daß eine Retro-Aldol-Spaltung eintritt.

EP 0 272 667 A2

## Verfahren zur Herstellung der Desmalonylverbindung von Makrolid-Lactonen

Makrolid-Lactone mit Malonsäurehalbester, wie z. B. Niphimycin, Guanidylfungin A, Copiamycin oder Azalomycin $F_4$ zeigen nach Abspaltung der Halbester eine deutlich höhere antifungische Wirkung. Die Desmalonylverbindungen sind fungizid, während die Ausgangsverbindungen nur fungistatisch wirken [K. Takesako, J. Antibiot. 38, 1363 (1985); K. Takesako, J. Antibiot. 29, 713 (1986)]. Die Wasserlöslichkeit der Desmalonylverbindungen ist ebenfalls verbessert.

Die direkte Hydrolyse des Malonsäurehalbester-Makrolids zur entsprechenden Desmalonylverbindung galt bislang als nicht möglich. Aufgrund der Hydroxylgruppe in β-Stellung zur Hemiketalgruppe im Sechsring des Niphimycin wird beispielsweise bei direkter Hydrolyse eine Retro-Aldol-Spaltung durchgeführt. Damit entsteht ein Gemisch mehrerer Komponenten, die bisher noch nicht aufgetrennt werden konnten [J. W. Westley, J. Chem. Soc. Chem. Commun. 71 (1970); K. Takasako, J. Antibiot. 28, 1363, (1983), W. Keller-Schierlein, Helv. Chim. Acta 66, 226 (1983)].

Nach der Alkylierung der genannten Hemiketalgruppe in den aufgeführten Makrolid-Lactonen ist zwar die Abspaltung der Malonsäuregruppen möglich. Es ist jedoch bekannt, daß derartige Alkylmakrolide eine schlechtere biologische Wirkung zeigen [K. Takesako, J. Antibiot. 38, 1363 (1985) und 29, 713 (1986)].

Überraschend wurde nun gefunden, daß in Gegenwart einer Base unter geeigneten Bedingungen eine direkte hydrolytische Abspaltung der Malonsäuregruppen ohne Retro-Aldol-Spaltung möglich ist.

Die Erfindung betrifft somit ein Verfahren zur Herstellung der Desmalonylverbindung von Makrolid-Lactonen, das dadurch gekennzeichnet ist, daß die Verbindung der allgemeinen Formel I,

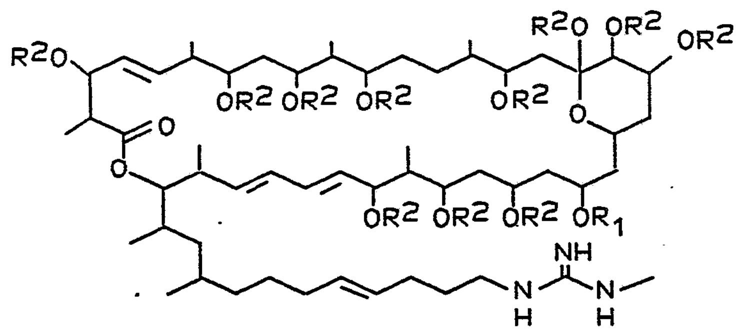

I

in der $R^1$ und $R^2$ unabhängig voneinander Wasserstoff oder eine Malonylgruppe ist, wobei höchstens ein $R^2$-Substituent eine Malonylgruppe darstellt, ausgenommen die Verbindung der Formel, in der $R^1$ und alle $R^2$ gleichzeitig Wasserstoff sind, in Gegenwart einer Base hydrolysiert wird.

Im folgenden wird die Erfindung im einzelnen, insbesondere in den bevorzugten Ausführungsformen, beschrieben. Die Erfindung wird ferner in den Patentansprüchen definiert.

Die Verbindung der allgemeinen Formel I wird in einem geeigneten Lösungsmittel gelöst. Beispielsweise können polare Lösungsmittel, wie Wasser, Alkohole, oder deren Mischungen verwendet werden. Bevorzugte Lösungsmittel sind niedere Alkohole, wie beispielsweise Methanol, Ethanol, n-Propanol, iso-Propanol und n-, iso-oder tertiär Butanol.

Die Lösungen werden mit geeigneten Basen versetzt. Geeignet sind Amine, Metallhydroxide, wie z. B. Erdalkali-oder Alkalihydroxide, wobei Natrium-oder Kaliumhydroxid aus der letzteren Gruppe wiederum bevorzugt sind. Gute Erfolge wurden auch bei der Verwendung von Alkoholaten als Basen erzielt, insbesondere mit Erdalkali-und Alkalialkoholaten niederer Alkohole, wobei Natrium-und Kaliumalkoholate besonders bevorzugt sind. Wenn vorher die zu hydrolysierende Verbindung in einem Alkohol gelöst wird, verwendet man zur Hydrolyse zweckmäßig das Alkoholat dieser Alkohole.

Die Reaktion kann bei Temperaturen von -30°C bis +60°C stattfinden. Bevorzugt kommt jedoch ein Temperaturbereich von 0° bis 40°C, insbesondere Raumtemperatur, zur Anwendung. Je nach Reaktionstemperatur wird das Gemisch aus zu hydrolysierender Verbindung und Base unterschiedlich lang inkubiert. Vorteilhaft hat sich eine Inkubationszeit von ca. 5 bis 60 Stunden, insbesondere 24 Stunden, erwiesen.

Mit Hilfe der Reaktionszeit kann man die Zahl der Malonylgruppen steuern, die abgespalten werden sollen. So kann beispielsweise unter oben genannten Bedingungen bei einer Reaktionszeit von ca. 1 bis 3 Stunden aus dem Amycin (2 Malonylgruppen) das Niphimycin (1 Malonylgruppe) hergestellt werden.

Der anschließende Verwendungszweck entscheidet, ob die hydrolysierte Verbindung isoliert wird. Zur Isolierung stellt man den pH-Wert des Reaktionsgemisches auf einen Wert im Bereich von 6 bis 8 ein. Nach

dem Abdestillieren des Lösungsmittels wird die Verbindung durch Auftrennung and geeigneten Adsorptionsmitteln, z. B. Kieselgel, Aluminiumoxid etc., und Elution mit organischen polaren Lösungsmitteln oder deren Gemischen gereinigt.

In den folgenden Beispielen wird die Erfindung detailliert erläutert. Prozentangaben beziehen sich auf das Gewicht, wenn nicht anders angegeben.

Beispiele:

1. Herstellung der Desmalonylverbindung

a. 1 g Amycin (0,81 mmol) werden in 75 ml Methanol mit 100 mg Natriumhydrid (50 % in Ölsuspension) 24 Stunden bei 20°C gerührt. Nach der Neutralisation mit 5 N Salzsäure wird der Alkohol im Vakuum abdestilliert. Der erhaltene Sirup wird an 100 g Kieselgel mit Essigester/Methanol/Wasser (15:2:1, 800 ml und 8:2:1; v:v:v) chromatographiert. Kristallisation aus Essigester/Methanol ergibt 770 mg (90 %) Desmalonylverbindung, in der $R^1$ und alle $R^2$ Wasserstoff bedeutet.

b. 1 g Niphimycin (0,87 mmol) werden in 50 ml Methanol mit 60 mg Natriumhydrid (50 % in Ölsuspension) 24 h bei 20°C gerührt. Nach der Neutralisation mit 5 N Salzsäure wird wie in in Beispiel 1 beschrieben, aufgearbeitet. Es werden 808 mg (88 %) Desmalonylverbindung erhalten, in der $R^1$ und alle $R^2$ Wasserstoff bedeutet.

c. Man verfährt gemäß Beispiel 1, beendet die Reaktion jedoch nach 2 Stunden, so erhält man 920 mg (93%) Niphimycin.

2. Verwendung der Desmalonylverbindung als antimykotisches Agens

Die in vitro-Prüfungen auf antimykotische Wirkung wird im Reihenverdünnungstest an Hefepilzen (Candida albicans) und Schimmelpilzen (Aspergillus niger) durchgeführt [Material und Methodik: Mikrotitrationstechnik veröffentlicht in Mykosen 27, 14 (1984)]. Wie aus der Tabelle A ersichtlich, zeigt bei diesen in vitro-Prüfungen die erfindungsgemäß hergestellte Desmalonylverbindung, in der $R^1$ und alle $R^2$ Wasserstoff bedeutet, gute antimykotische Eigenschaften, die denjenigen der Referenzsubstanz Niphimycin überlegen sind.

| Präparat | Minimale Hemmkonzentration (µg/ml) gegen | |
|---|---|---|
| | Candida albicans | Aspergillus niger |
| Niphimycin | 7,8 | 3,9 |
| Desmalonylverbindung | 3,9 | 1,95 |

**Ansprüche**

1. Verfahren zur Herstellung der Desmalonylverbindung von Makrolid-Lactonen, dadurch gekennzeichnet, daß die Verbindung der allgemeinen Formel I,

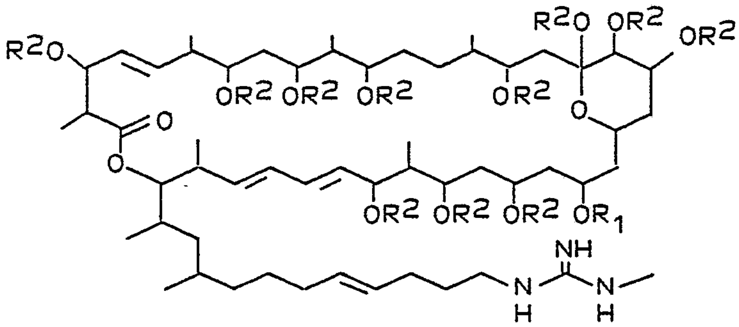

I

in der $R^1$ und $R^2$ unabhängig voneinander Wasserstoff oder eine Malonylgruppe ist, wobei höchstens ein $R^2$-Substituent eine Malonylgruppe darstellt, ausgenommen die Verbindung der Formel, in der $R^1$ und alle $R^2$ gleichzeitig Wasserstoff sind,

in Gegenwart einer Base hydrolysiert wird.

2. Verfahren nach Anspruch 1, dadurch gekennzeichnet, daß als Basen Amine, Alkali-oder Erdalkalihydroxide und/oder Alkali-oder Erdalkalialkoholate eingesetzt werden.

3. Verfahren nach Anspruch 2, dadurch gekennzeichnet, daß als Basen Natrium-oder Kaliumhydroxide und/oder Natrium oder Kaliumalkoholate niederer Alkohole eingesetzt werden.

4. Verfahren nach einem oder mehreren der Ansprüche 1 bis 3, dadurch gekennzeichnet, daß die Reaktion in einem niederen Alkohol als Lösungsmittel durchgeführt wird.

5. Verfahren nach einem oder mehreren der Ansprüche 1 bis 4, dadurch gekennzeichnet, daß die Reaktion bei -30°C bis +60°C durchgeführt wird.

6. Verfahren nach Anspruch 5, dadurch gekennzeichnet, daß die Reaktion bei 0 bis 40°C durchgeführt wird.

7. Verfahren nach Anspruch 6, dadurch gekennzeichnet, daß die Reaktion bei Raumtemperatur durchgeführt wird.

8. Verfahren nach einem oder mehreren der Ansprüche 1 bis 7, dadurch gekennzeichnet, daß das Reaktionsgemisch über einen Zeitraum von 5 bis 60 Stunden inkubiert wird.